# EUROPEAN PATENT SPECIFICATION

(11) **EP 1 505 956 B1**
(45) Date of publication and mention of the grant of the patent: **14.02.2007**
(21) Application number: 03730190.0
(22) Date of filing: 29.04.2003
(51) Int. Cl.: A61K 9/20

(54) **A METHOD TO IMPROVE SURFACE PROPERTIES OF PHARMACEUTICAL TABLETS**
METHODE ZUR VERBESSERUNG DER OBERFLÄCHENEIGENSCHAFTEN VON TABLETTEN
PROCEDE PERMETTANT D'AMELIORER LES PROPRIETES DE SURFACE DES COMPRIMES PHARMACEUTIQUES

(30) Priority: 07.05.2002 EP 02076837
(43) Date of publication of application: 16.02.2005
(73) Proprietor: Fabre-Kramer Pharmaceuticals, Inc., Houston, TX 77057 (US)
(72) Inventor: DE HAAN, Pieter, NL-5340 BH Oss (NL); GRINTJES, Lambertus, Pedro, Everhardus, NL-5340 BH Oss (NL); VAN LARE, Coert, Elisabeth, Johannes, NL-5340 BH Oss (NL)
(74) Representative: Moutard, Pascal Jean
(86) International application number: PCT/EP2003/050134
(87) International publication number: WO 2003/094896

(56) References cited:
- EP-A- 1 121 931
- US-A- 4 919 938

## Description

The invention relates to a method to improve surface properties of tablets having a matrix consisting of at least 55% of a cellulose ether.

There are a number of different purposes of modifying the surface properties of pharmaceutical tablets. It is usually done by coating the tablets (see US 4 919 938). The purpose may be to influence the release pattern of the active ingredient, to influence the location in the gastro-intestinal system where the formulation is degraded or the active ingredient is released. Also, a coating may have the function to improve the surface properties for purposes of improved appearance, for ease of handling or reducing dust formation during handling or in order to mask the taste of certain ingredients. Coating techniques have a long history and have acquired many elaborations over time (Gennaro *et al,* Remington; The Science and Practice of Pharmacy; 20th ed., Publisher: Lippincott Williams & Wilkins; Baltimore; USA; Chapter 46: Coating of pharmaceutical dosage forms). It is therefore all the more surprising that a whole new and extremely simple principle of tablet surface treatment can still be added to the art. For the purpose of reducing dust formation during tablet processing, transportation and handling of the tablets and packaged tablets by the user, it has now been found that surface properties of tablets, having a matrix consisting of at least 55% of a cellulose ether, can be improved by briefly exposing the tablets to a hydrous solvent.

When manufacturing and packaging the tablets and transporting the tablets in the package, those tablets having a matrix consisting of at least 55% of a cellulose ether are losing particles either by attrition, which is wear of the tablets due to contact against other material, or by abrasion, which is wear of the tablets due to contact among the tablets. Tablets of the defined characteristics are prepared by dry-mixing processes without the use of granulation liquids. Thus far, such a problem was in general solved by treating the tablet surface with coating materials and thereby adding a layer of another composition to the tablets. With the newly invented method the surface properties are changed but the change is not caused by the addition of other material. The tablet surface is exposed to solvent which is also removed during the process. The effect can preferrably be obtained by spraying the tablets with the solvent. With spraying techniques the solvent is made to evaporate rapidly after contacting the tablet surface. During this brief contact of the solvent with the surface, the surface changes to a surface with reduced loss of small particles. Also, the rougher surface of untreated tablets gives coloured tablets a more matted appearance, which becomes less so after spraying and drying with a hydrous solvent.

A method to improve surface properties of tablets is understood to be a process resulting in the formation of a surface layer on the tablets in order to change its surface properties. The changed surface resulting from the method according to the invention is not necessarily caused by the formation of a layer of different chemical composition, since it may have resulted from changing the physical properties of the outer layer of the tablets by temporary exposure to the hydrous solvent. This may have caused temporarily the formation of a gel-type layer, which, after evaporation of the solvent, could have been transformed into a thin layer with the desired properties. One may speculate that the newly formed structure of the layer causes the effects of the invention.

Tablets having a matrix consisting of at least 55% of a cellulose ether are typically tablets with the cumbersome dust forming surface if not treated with the method according to the invention. It is the high content and the properties of the carrier which cause the tablets having such a surface. Cellulose ethers are used as carrier in dry-mix tablets, as binder in wet-granulation and can be used in coating techniques as film-forming polymers.

Cellulose ethers are carriers, which are gel-forming with water. Such carriers tend to retain other ingredients for a longer time upon absorption of water in the outer layer and consequently are suitable for extended release formulation. Examples of such carriers can be found in the group of hydroxy-(1C-3C)alkyl(1C-3C)alkylcelluloses, such as hydroxymethylcellulose, hydroxyethylcellulose and the preferred hydroxypropylmethylcellulose. Other gel-forming carriers can be found in the standard compilation of pharmaceutically acceptable carriers and excipients, the Handbook of Pharmaceutical Excipients (3^{nd} edition edited by Arthur H. Kibbe; Published by the American Pharmaceutical Association, Washington D.C. and The Pharmaceutical Press, London in 2000). The cellulose ethers and the relevant theoretical views on their properties are discussed in Alderman, A., A review of cellulose ethers in hydrophilic matrices for oral controlled-release dosage forms, Int. J. Pharm. Tech. & Prod. Mfr., Vol 5, pages 1-9, 1984.

Briefly exposing the tablets to a hydrous solvent can be done in various manners with techniques commonly used in the art of pharmaceutical sciences (see for example the techniques described in the above cited textbook Remington, the Science and Practice of Pharmacy). A preferred method is spraying. During this process the tablets are typically exposed to the solvent during short periods of time. The solvent is sprayed on the tablets as droplets, which evaporate rapidly after having impacted the surface of the tablets. The conditions during spraying are set in such a way that the solvent is removed from the surface of the tablets before the surface becomes totally wetted and sticky. When too much solvent remains for too long a time on the surface of the tablets, these would deform or damage the surface excessively. It is a matter of optimizing the parts of the equipment and processing parameters to obtain satisfying results. It is entirely within the experience of the skilled person to optimize the process, once the purpose of the process, that is wetting the surface of the tablets for a brief period of time in order to modify the surface to the extent that dust-formation is reduced, is known An instructive text explaining the parameters involved in the analogous wet coating techniques is the contribution by Porter, Bruno and Jackson on page 82-109 of the handbook by Lieberman, H.A. and Lachman (Eds), L., Parmaceutical dosage forms, Tablets, Volume 3. The effect of the invention to be achieved, that is reduced dust formation by handling the tablets, can be assessed by methods available in the art. The effect can already be observed by visual observation of dust formation and by tactile inspection of the tablets, but more precise measurements can be made in standardised friability/abrasion tests.

A hydrous solvent is water or an equivalent polair, hydrogen-bonding solvent or mixtures of such solvents. Water and mixtures of water and ethyl alcohol are the most commonly used examples of these solvents. It is, of course, intended to use only such solvents as are accepted in pharmacy (See Kibbe, 2000; op. cit.)

Since the problem of dust formation with tablets having a matrix consisting of a cellulose ether are more serious depending on the nature and amounts of other constituents, such as the filler or binder and active ingredient, the method according to the present invention is preferrably to be applied to tablets having a matrix which consists of at least 65% of the cellulose ether and more preferrably of from 70 to 85 wt % of the cellulose ether. In particular an amount of carbohydrate binder from 7 to 10 wt % can be problematic for untreated tablets. Carbohydrate binders are for example cellulose (microcrystalline cellulose, such as Avicel pH 101), sugars, starches, amylopectin, dextrin, maltodextrin, gums and alginates.

The tablets for the method according to the present invention can have a total weight of at most 450 mg and may have a high relative amount of the active ingredient gepirone HCl (up to 13 to 21 wt % of the tablets) over the cellulosic polymer matrix material and also over the carbohydrate binder. With the present invention such high content tablets could still be made with sufficient stability during later handling.

Tablets obtained by the methods according to the invention are new in the art and are considered to be embodiments of the present invention.

### Example

### Tablets

| Tablets were made with the following composition | | |
|---|---|---|
| ingredients | mg | Function |
| Gepirone HCl | 80.0 | Active principle |
| Hydroxypropyl | 290.0 | Drug release controlling |
| Methylcellulose (Methocel | | polymer |
| K100M, Premium) | | |
| Microcrystalline Cellulose | 33.7 | Diluent/binder |
| (Avicel pH 101) | | |
| Euroxide yellow and red | 3.5 | Colorant |
| (E7055 and E7016) | | |
| Colloidal silicon dioxide | 1.6 | Glidant |
| (cab-o-sil M5) | | |
| Magnesium stearate NF | 1.2 | Lubricant |

### Method of manufacture

### Active pre-mixture:

Transfer the colloidal silicon dioxide, colorant (Euroxide yellow E 7055 and Euroxide Red E 7016), gepirone HCI powder and 20% of hydroxypropyl methylcellulose in 2 cu. Ft. planetary mixer (Hobart mixer). Mix ingredients for 15 minutes in a planetary mixer (Hobart Mixer). Label as 'Active Pre-Mix'.

### Blend for slugging

Mill the Active Pre-Mix in a Fitzmill using a perforated plate No. 0020 at high speed, impact forward to deagglomerate lumps, if any.

Transfer the Active Pre-Mix in a 10 cu. Ft. "V"-blender without an I-bar, while passing through #12 mesh screen and transfer the balance of 80% HPMC, microcrystalline cellulose and 50% of magnesium stearate, in the V-blender without an I-bar. Blend ingredients in the V-blender for 24 minutes and label as "Blend for Slugging".

### Slugging

Compress the blend into slugs using 7/8" round flat face bevelled plain tooling using a rotary Kikusui-Hercules compression machine.
In process controls:

| | |
|---|---|
| Weight: | 2250 mg |
| Hardness: | 7 kp |
| Targeted thickness: | 0.255" |

### Final Blend

Mill the slugs in an S.S. Fitzmill with screw feeder using a perforated plate No. 0093 at medium speed, knives forward. Transfer the milled mass into a 10 cu. Ft. V-blender without I-bar. Screen the balance of 50% magnesium stearate, through #18 mesh and transfer also into the V-blender. Blend for 6 minutes.

Compress tablets with a rotary Kikusui-Libra compression machine using 0.338" X 0.405" Ovoid rectangular dies.
In process controls:

| | |
|---|---|
| Run Weight, mg | 410 ± 29 |
| Hardness, kp | 20 ± 8 |
| Thickness, inches | 0.235 to 0.265 |

Store in tight containers until further coating.

### Surface treatment.

An amount of 7 kg tablets was sprayed with 1500 ml water in a Glatt coater. The coating device was pre-heated (temperature incoming air 60°C to 65°C) after which the coating pan was filled with tablets. Outgoing air temperature was set at 40°C, air volume at 300 m³/hour. During the initial warming of the tablets (1 minute) and first 10 minutes of coating, a low rotation speed (6 rpm) was used to reduce the wear/attrition of the tablets. After 10 minutes the rotation speed was increased to 12 ½ rpm and after coating the tablets were dried during 1 minute (total process time approx. 70 minutes; spraying rate 20 to 25 ml/min.). The surface treatment process was observed visually. Samples were taken every 10 minutes.

### Test methods for dust formation.

A) Visual observation
B) Mechanical testing of 10 tablets per plastic container:
   Settings vibrating table: 200 rpm, horizontal amplitude: 45 mm, test time: 45 minutes. Determination of mass loss by weighing. Before weighing, the tablets were cleaned by vacuum.

### Results

### Visual observation

Untreated tablets clearly show dust formation in contrast to water treated tablets. Tablets prepared without the water spraying surface treatment process are red/pink coloured. The water-treated tablets were darkened and the colour is more intense red. The colour of the water-treated tablets remains constant, even with the 70 minutes treatment and subsequent 2 hours drying at 50°C in a stove. A water-treated tablet was cut and a darker coloured outer layer could clearly be seen.

### Dust formation measurements.

The following table gives the average amount (n=3) of dust formed during testing.

| Treatment | Average dust formation (n=3, %) |
|---|---|
| Untreated tablets | 3.04 |
| Water treated (10 minutes) with drying | 0.03 |
| Water treated (70 minutes) with drying | 0.03 |
| Water treated (70 minutes with additonal drying for 2 hours at 50°C) | 0.04 |

It is clear from this table that dust formation is significantly reduced by spraying with water only, even after 10 minutes spraying, after which a total amount of 200 ml water was sprayed on 7 kg tablets.

## Claims

1. A method to improve surface properties of tablets having a matrix consisting of at least 55% of a cellulose ether, **characterised in that** the method comprises briefly exposing the tablets to a hydrous solvent.

2. The method according to claim 1, **characterised in that** the matrix is consists of at least 65% of the cellulose ether.

3. The method according to claim 2, **characterised in that** the matrix consists of from 70 to 85 wt % of the cellulose ether.

4. The method according the anyone of claim 1-3, **characterised in that** the tablets further comprise an amount of carbohydrate binder from 7 to 10 wt %.

5. A tablet obtained by any one of the method according to claim 1-4.

## Patentansprüche

1. Verfahren zum Verbessern der Oberflächeneigenschaften von Tabletten, welche eine Matrix, bestehend aus wenigstens 55% eines Celluloseethers aufweisen, **dadurch gekennzeichnet, dass** das Verfahren das kurzzeitige Behandeln der Tabletten mit einem wasserhaltigen Lösungsmittel umfasst.

2. Das Verfahren nach Anspruch 1, **dadurch gekennzeichnet, dass** die Matrix aus wenigstens 65% des Celluloseethers besteht.

3. Das Verfahren nach Anspruch 2, **dadurch gekennzeichnet, dass** die Matrix wenigstens von 70-85 Gew.-% des Celluloseethers besteht.

4. Das Verfahren nach irgendeinem der Ansprüche 1 bis 3, **dadurch gekennzeichnet, dass** die Tabletten ferner einen Kohlenhydratbindemittelanteil von 7 bis 10 Gew.-% enthalten.

5. Eine Tablette, erhalten durch irgendeines der Verfahren gemäß den Ansprüchen 1 bis 4.

## Revendications

1. Procédé pour améliorer les propriétés de surface de comprimés, ayant une matrice consistant en au moins 55% d'un éther de cellulose, **caractérisé en ce que** le procédé comprend la brève exposition des comprimés à un solvant hydraté.

2. Procédé selon la revendication 1, **caractérisé en ce que** la matrice consiste en au moins 65% de l'éther de cellulose.

3. Procédé selon la revendication 2, **caractérisé en ce que** la matrice consiste en 70 à 85% en poids de l'éther de cellulose.

4. Procédé selon l'une quelconque des revendications 1-3, **caractérisé en ce que** les comprimés comprennent en outre, une quantité de liant hydrate de carbone, allant de 7 à 10% en poids.

5. Comprimé obtenu par l'un quelconque du procédé selon les revendications 1-4.
